# EUROPEAN PATENT SPECIFICATION

(11) **EP 1 377 190 B1**
(45) Date of publication and mention of the grant of the patent: **03.08.2005**
(21) Application number: 02728735.8
(22) Date of filing: 11.04.2002
(51) Int. Cl.: A47C 7/46

(54) **LUMBAR SUPPORT DEVICE**
LENDENSTÜTZVORRICHTUNG
DISPOSITIF DE SUPPORT LOMBAIRE

(30) Priority: 11.04.2001 US 832692
(43) Date of publication of application: 07.01.2004
(73) Proprietor: L & P Property Management Company, South Gate, CA 90280 (US)
(72) Inventor: MUNDELL, Donald, David, Carthage, MO 64836-3343 (US)
(74) Representative: Banzer, Hans-Jörg, Dipl.-Ing.
(86) International application number: PCT/US2002/011399
(87) International publication number: WO 2002/083029

(56) References cited:
- US-A- 3 095 188
- US-A- 3 241 879
- US-A- 3 378 299
- US-A- 4 019 777
- US-A- 4 148 522
- US-A- 4 714 291
- US-A- 4 715 653
- US-A- 5 954 399
- US-A- 6 068 336
- US-A- 6 152 531

## Description

The present invention relates generally to lumbar support devices. More particularly, the present invention relates to lumbar support devices that are capable of changing shape, especially curvature in the lumbar region.

### Background of the Invention

Lumbar support devices have been integrated into seats to change their shape, thereby allowing each occupant to adjust the support provided by the seat. The curvature of these devices is traditionally adjustable so that an occupant can operate the device to push the seat forward towards the occupant's spinal column in the lumbar region. It is generally known to change the curvature of a lumbar support device using an actuator assembly that moves a support structure, such as a sinusoidal spring element. It is also well known to provide an actuator assembly that is either manually operated, using a handle or knob, or power assisted, using a drive motor and control switches. Increased curvature is usually accomplished by moving the support structure forward into the lumbar region, rotating sections of the support structure into the lumbar region, or bowing the support structure out into the lumbar region.

From US 4 714 291, a lumbar support device is known which uses a lever type mechanism for moving a lumbar plate. For this purpose an anchor member connects at one end to the lumbar plate and is at the other end rotatably mounted via a pin which is passed through a ring shaped pivot portion. A bent portion extends from the pivot portion and is engaged with an actuating mechanism. Operation of the actuating mechanism results in a movement of the lumbar plate generally perpendicular to its support plane.

### Summary of the Invention

The present invention provides a lumbar support device according to independent claim 1. The dependent claims define preferred and advantageous embodiments of the invention.

One embodiment of the present invention relates to a strap lumbar device having a seat frame, a pair of brackets connected to the seat frame, a spring assembly connected to the pair of brackets in such a manner as to form a center section traversing the seat frame and a pair of cantilevered ends on opposite sides of the center section, and an actuator

assembly operatively connecting the cantilevered ends. The pair of brackets respectively provide a pair of fulcrums about which the cantilevered ends can rotate.

In the preferred embodiment, the spring assembly has two integrally-formed, sinusoidal spring elements attached by a connector, and the center section of the spring assembly has a recessed portion. In other embodiments of the invention, a single sinusoidal spring has only one cantilevered end that rotates about a bracket, and a leaf spring is cantilevered about a pair of coil springs. The lumbar support device can be oriented horizontally or vertically in the seat frame, and the orientation of the cantilevered ends can be reversed.

In operation, the fulcrums allow the cantilevered ends to function as levers. The actuator assembly moves the levers which rotate about the fulcrums and bow the center section. The fulcrums are located between the distal end of the levers and the center section of the spring to stop the lever from sliding in the bracket and force the lever to rotate about the bracket.

Another embodiment of the invention pertains to a lumbar support device comprising first and second mounts, a main body, and an actuator assembly. The mounts are spaced apart from each other and the main body has opposite end margins that are operatively connected to the mounts. The main body consists essentially of wire, and further comprises an intermediate portion that extends along the longitudinal trajectory of the main body between the first and second mounts. A point along the longitudinal trajectory defines first and second sub-portions of the intermediate portion and the wire is shaped and configured such that the first sub-portion has an average bending stiffness that is less than that of the second sub-portion. Additionally, the first end margin of the main body comprises a lever arm that extends in a cantilever manner and an actuator assembly is operatively connected to the main body via the lever arm. The actuator assembly is configured and adapted to induce a bending moment in the first end margin of the main body via the lever arm such that the longitudinal trajectory of the main body is selectively bendable between first and second positions. Due at least partially to the difference between the average bending stiffness of the first sub-portion and the average bending stiffness of the second sub-portion, the rate of curvature change of the longitudinal trajectory along the first sub-portion is greater than the rate of curvature change of the longitudinal trajectory along the second sub-portion. In turn, this results in the curvature of the first sub-portion increases substantially more than that of the second sub-portion in response to the bending moment provided by the actuator assembly. Thus, the first sub-portion of the main body tends to bulge more than the second sub-portion when the longitudinal trajectory of the main body is selectively bent between first and second positions, thereby providing the main body with a desirable shape in an efficient manner. Furthermore, the fact that main body has such properties, and yet consists essentially of wire, allows the lumbar support device to be manufactured with low costs and simplifies the assembly of the lumbar support device.

In yet another embodiment of the invention, a lumbar support device comprises first and second mounts, a main body, and an actuator assembly. The mounts are spaced apart from each other and the main body has opposite end margins that are operatively connected to the mounts. The first end margin comprises a lever arm that is formed by the wire that forms the main body. The lever arm extends in a cantilever manner and the actuator assembly is operatively connected to the main body via the lever arm in a manner such that the actuator assembly is capable of inducing an increasing bending moment to the first end margin. By forming the lever arm out of the wire that also forms the main body, the assembly procedure of the lumbar support device is simplified and the costs of the lumbar support device is substantially reduced.

In yet the following, a method of adjusting the contour of a seatback of a seat assembly is disclosed. The method comprises providing a seat assembly having a rigid seat frame and a seatback and operatively attaching first and second mounts to the seat frame in a manner such that the first and second mounts are spaced apart from each other adjacent the seatback. The method further comprises providing a main body that consists essentially of wire for supporting the seatback and for providing the seatback with a contour. The main body has first and second end margins and the wire is shaped and configured such that a first portion of the main body that extends along one of two contiguous halves of the longitudinal trajectory of the main body has a bending stiffness that is substantially less than a bending stiffness of a second portion of the main body that extends along the other of the two halves. The method yet further comprises operatively connecting the first end margin of the main body to the first mount and the second end margin to the second mount in a manner such that the main body supports the seatback and operatively connecting an actuator assembly to the main body. The actuator assembly is configured and adapted to selectively induce an increasing bending moment to at least one of the end margins of the main body such that the longitudinal trajectory of the main body increases in curvature in response to the increasing bending moment. The method yet further comprises adjusting the curvature of the main body from a first position to a second position via inducing an increasing bending moment to the at least one of the first and second end margins of the main body using the actuator assembly. The adjusting of the curvature of the main body causes the first portion of the main body to increase in curvature a greater amount than that of the second portion of the main body as a result of the bending stiffness of the first portion of the main body being substantially less than that of the second portion of the main body. This results in the contour of the seatback being adjusted in a desirable manner.

It is a purpose of the present invention to provide a lumbar support device that is simple and affordable to manufacture.

It is a further purpose of the present invention to provide a mechanically simplified lumbar support device that has a thin profile when flat.

Further advantages of the present invention will be apparent from the description below with reference to the accompanying drawings in which like numbers indicate like elements.

### Brief Description of the Drawings

FIG. 1 illustrates the preferred embodiment of the lumbar support device installed in a frame.
FIG. 2 illustrates a front view of the preferred embodiment of the lumbar support device.
FIG. 3 illustrates a top view of the preferred embodiment of the lumbar support device.
FIG. 4 illustrates a front view of a first alternative embodiment of the lumbar support device according to the present invention.
FIG. 5 illustrates a top view of the first alternative embodiment of the lumbar support device illustrated in FIG. 4.
FIG. 6 illustrates a second alternative embodiment of the lumbar support device according to the present invention.
FIG. 7 illustrates a third alternative embodiment of the lumbar support device according to the present invention.
FIG. 8 illustrates a fourth alternative embodiment of a lumbar support device installed in a frame.
FIG. 9 illustrates a side view of the fourth alternative embodiment of the lumbar support device illustrated in FIG. 8.
FIG. 10 illustrates a fifth alternative embodiment of a lumbar support device according to the present invention.
FIG. 11 illustrates a sixth alternative embodiment of a lumbar support device according to the present invention.

### Detailed Description of the Invention

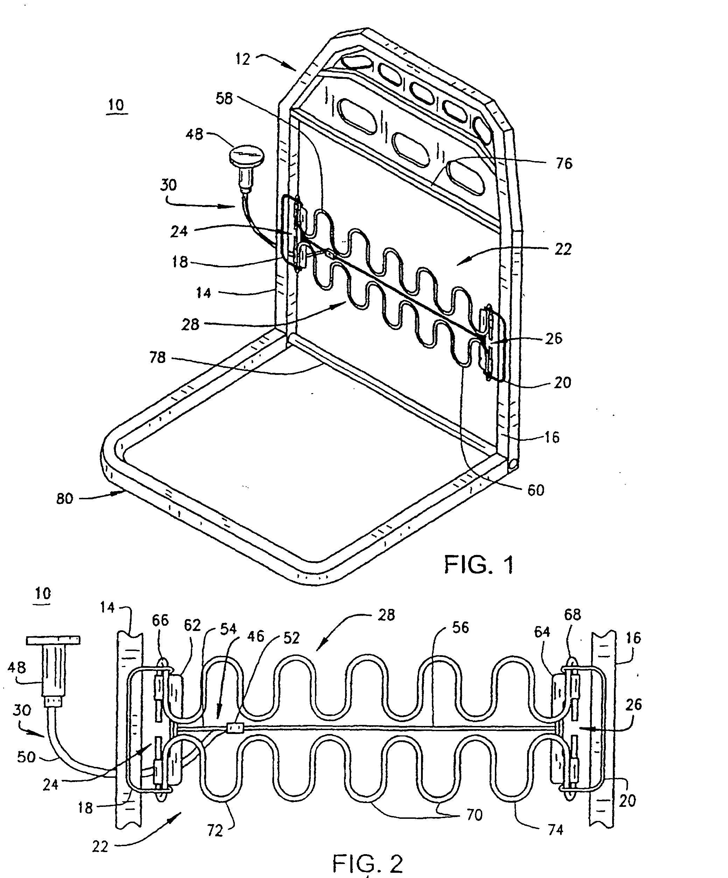

As shown in FIGS. 1, 2 and 3, the preferred embodiment of a lumbar support device 10 generally includes a seat frame 12 having a first side 14 and a second side 16, a first bracket 18 and a second bracket 20 respectively fixed to the first and second sides 14, 16, a spring assembly 22 connected to the pair of brackets 18, 20 in such a manner as to respectively form first and second cantilevered ends 24, 26 on opposite sides of a center section 28, and an actuator assembly 30 operatively connecting the cantilevered ends 24, 26. The first and second brackets have first and second fulcrums 32, 34, respectively. The first fulcrum 32 is located proximately to the first side 14 and located distally from the second side 16, and the second fulcrum 34 is located proximately to the second side 16 and located distally from the first side 14.

In the preferred embodiment, the center section 28 of the spring assembly 22 is integrally, formed with the first and second cantilevered ends 24, 26. The first and second cantilevered ends 24, 26 are rotatably connected to and cantilevered about the first and second brackets 18, 20, respectively, and the center section 28 traverses the seat frame between the first and second brackets 18, 20. The first and second cantilevered ends 24, 26 have first and second distal ends 36, 38, respectively, such that the first and second cantilevered ends 24, 26 define first and second levers 40, 42, respectively. The first lever 40 extends from the first fulcrum 32 to the first distal end 36, and the second lever 42 extends from the second fulcrum 34 to the second distal end 38. The actuator assembly 30 connects the first and second distal ends 36, 38 and operates to force the distal ends 36, 38 of the respective levers 40, 42 toward each other, thereby rotating the levers 40, 42 about the first and second fulcrums 32, 34, respectively, and bowing the center section 28. In the drawings, broken lines illustrate the actuated position.

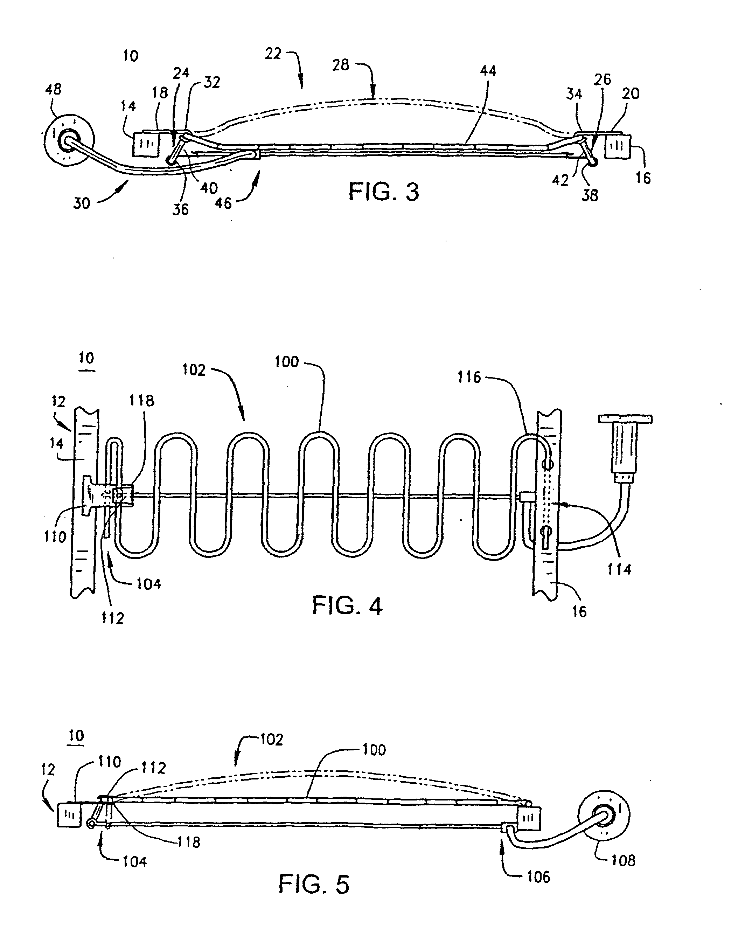

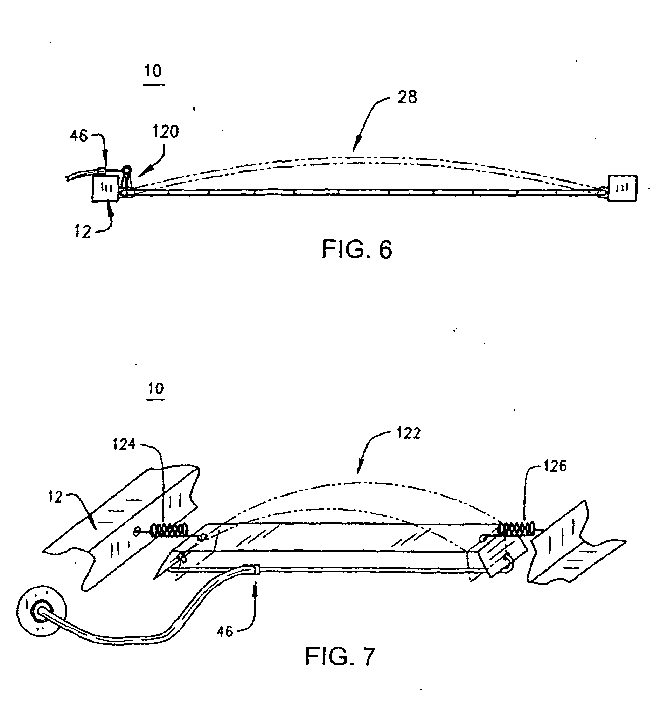

The center section 28 has a recessed portion 44 that reduces the thickness 46 of the center section 28 when not bowed. The brackets 18, 20 are rigidly attached to the seat frame 12 and the cantilevered ends 24, 26 are attached to their respective fulcrums 18, 20 such that they are only able to rotate. The fulcrums 18, 20 stop the respective cantilevered ends 24, 26 from sliding. Without the recessed portion 44, a straight center section (see FIGS. 5 & 6) would have the same length as a straight line between the brackets 18, 20 and forcing curvature in the straight center section would bow the center section, requiring the center section to extend and requiring the actuator assembly to provide additional force to produce both curvature and extension. With the recessed portion 44, the center section 28 has a greater length than the straight line between the brackets. Therefore, the recessed portion 44 reduces the force needed by the actuator assembly 30 to bow the center section 28 because less force, if any, is necessary to extend the center section 28.

The actuator assembly 30 preferably includes a bowden cable assembly 46 and an actuator 48. The bowden cable assembly 46 has a sheathed section 50, a base 52, a rod 54 and an unsheathed section 56. The rod 54 and the unsheathed section 56 respectively link the distal ends 36, 38 of the levers 40, 42. The base 52 holds the rod 54 and one end of the sheathed section 50, and the other end of the sheathed section 50 is connected to the actuator 48. To force the distal ends 36, 38 of the respective levers 40, 42 toward each other, the actuator 48 transmits a tractive force through the bowden cable assembly 46 to the distal ends 36, 38. Although the preferred embodiment uses the tractive actuator assembly 30, other types of actuator assemblies, including those supplying pulsive forces may also be used. For example, as one type of pulsive actuator assembly, screw actuators (not shown) could engage threaded rods (not shown) to push the distal ends 36, 38 of the respective levers 40, 42 toward each other.

The spring assembly 22 is preferably formed from a pair of sinusoidal springs 58, 60 that are similarly attached to the brackets 18, 20. For each of the sinusoidal springs 58, 60, the center section 28 is integrally formed with the cantilevered ends 24, 26 from a single wire bent into the sinuous shape. The springs 58, 60 are held together by a pair of connectors 62, 64, but according to the present invention, either one of the pair, sinusoidal spring 58 or sinusoidal spring 60, could be used alone, as illustrated in FIGS. 4, 5 and 6. In the preferred embodiment, a first loop 66 is rotatably connected to and cantilevered about the first bracket 18, thereby defining the first cantilevered end 24, and a last loop 68 is rotatably connected to and cantilevered about the second bracket 20, thereby defining the second cantilevered end 26. The center section 28 has a plurality of loops 70 between the pair of brackets 18, 20, including a second loop 72 integrally formed with the first loop 66 and a second-to-last loop 74 integrally formed with the last loop 68.

As illustrated in the preferred embodiment, the first side 14 is generally opposite the second side 16, the first side 14 being on the right side of the seat frame 12 and the second side 16 being on the left side of the seat frame 12. The seat frame 12 also has a top side 76 and a bottom side 78 that can alternatively be used as the first side 14 and the second side 16, respectively. The present invention can also be mounted in reverse orientations, and the present invention may be attached to the bottom portion 80 of the seat frame 12.

As illustrated in FIGS. 1, 2 and 3, the first and second brackets 18, 20 are directly and rigidly attached to the first and second sides 14, 16, respectively. Such a fixed connection can be made by welding the brackets to the seat frame, by mounting the brackets with hardware, by integrally forming the brackets in the seat frame, or by using other methods to make a direct, rigid connection. Additionally, the connection between the brackets 18, 20 and the seat frame 12 does not necessarily need to be direct or rigid. An example of an indirect connection would be where an additional structural element is interposed between the brackets 18, 20 and the seat frame 12, such as a coil spring (not shown), in which case the connection would neither be direct nor rigid. Alternatively, the brackets 18, 20 may be directly connected to the seat frame 12 and the coil springs may be interposed between the fulcrums 32, 34 and the respective levers 40, 42. An example of a direct connection that is not rigid could be a rod having a loop (not shown) rotatably attached to the seat frame, such as the fulcrums 32, 34 of the brackets 18, 20, or a coil spring attached at one end to the seat frame and attached at its opposite end to the lever (see FIG. 7). Finally, the brackets can traverse the seat frame 12 in a direction substantially perpendicular to the center section 28 and still provide first and second fulcrums 32, 34 that are proximate and distal from the respective sides 14, 16. For example, in the configuration where the center section 28 horizontally traverses (between left and right) the seat frame 12, the brackets can be a pair of generally parallel rods (not shown) on opposite sides of the frame that are attached to the frame at the top side and the bottom side. With such a configuration, the rod traversing the frame on the left side could provide a fulcrum proximate to the left side and the rod traversing the frame on the right side could provide a fulcrum proximate to the right side.

An alternative embodiment of the lumbar support device 10 is illustrated in FIGS. 4 and 5. A sinusoidal spring 100 is similar to the spring assembly 22 described in the preferred embodiment, but the center section 102 traverses straight across the seat frame 12 without any recessed portion. The spring 100 only has one lever 104 at the first side 14 of the seat frame 12, and a bowden cable assembly 106 connects the lever 104 with an actuator 108. A bracket 110 is rigidly attached to the seat frame 12 at the first side 14. The bracket 110 has a hook 112 to hold the lever 104, thereby allowing the lever 104 to slide somewhat as well as rotate in the hook 112. An integral bracket 114 is formed in the second side 16 of the seat frame 12, and the second end 116 of the spring 100 is rotatably attached to the integral bracket 114.

As discussed above, without any recessed portion, the center section 102 has the same length as a straight line between the brackets 110, 116. The bracket 110 with the hook 112 allows the actuator 108 to bow the center section 102 with less force than would be necessary if the lever 104 is only permitted to rotate. For example, replacing the bracket 110 with a bracket with a loop, as shown in the preferred embodiment, would prevent the lever 104 from any sliding or translation, and the actuator 108 would force both curvature and extension in the center section 102. Although the lever 104 is allowed to slide in the bracket 114, sliding is limited because the bracket 114 has a fulcrum 118 that stops the lever 104 from sliding and forces the lever 104 to rotate.

FIGS. 6 and 7 illustrate other alternative embodiments of the present invention for the lumbar support device 10. FIG. 6 shows a lever 120 that is oriented opposite from the other embodiments. As with any of the embodiments, if the spring is formed with a curvature shape, the lever can be used in reverse to flatten the spring, in which case a tractive actuator assembly could be replaced with a pulsive actuator assembly and vice-versa. FIG. 7 shows a leaf spring 122 connected to the seat frame 12 through a pair of coil springs 124, 126 which serve as brackets. Although the preferred embodiment illustrated in FIGS. 1, 2 and 3 has a spring assembly 22 with pair of sinusoidal springs 58, 60 and a pair of cantilevered ends 24, 26, it is evident from the alternative embodiments that the lumbar support device 10 may have a single spring and a single cantilevered end, and that different types of springs will work. In each embodiment of the lumbar support device 10 discussed above, including the preferred embodiment, every fulcrum is preferably located between the distal end of the lever and the center section of the spring.

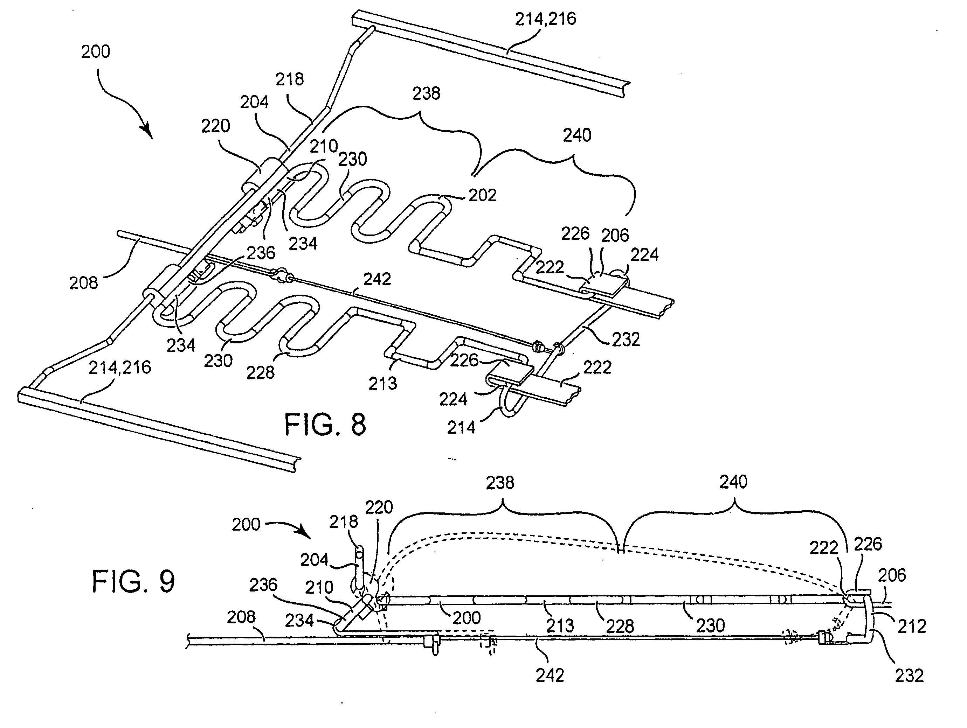

FIGS. 8 and 9 illustrate a fourth alternative embodiment of a lumbar support device 200 of the present invention. The lumbar support device 200 of the fourth embodiment comprises a main body 202 that is similar to the spring assembly described above, mounts 204, 206, and an actuator assembly 208. The main body 202 has first 210 and second 212 end margins and is formed essentially of wire. The wire is preferably metal spring wire of the type known in the art for use in seat assemblies. The first end margin 210 of the main body is preferably connected to the first mount 204, 206 and the second end margin 212 is preferably connected to the second mount such that an intermediate portion 213 of the main body extends longitudinally therebetween. As shown in FIG. 8, the mounts 204, 206 of the lumbar support device 200 are preferably attached to a seat frame 214 in a manner such that they are spaced vertically apart from each other and where they will position the main body 202 adjacent the seatback (not shown) of the seat in which the lumbar support device is installed. As such, the longitudinal dimension of main body 202 of the lumbar support device 200 extends generally parallel to the sides 216 of the seat frame 214.

The first mount 204 of the lumbar support device 200 of the fourth embodiment preferably comprises a single wire mount 218 that traverses the width of seat frame 214 from one of the seat frame sides 216 to the other. The first mount 204 also preferably comprises a connector 220 formed of glass reinforced nylon. The connector 220 is pivotally connected to the wire mount 218 and to the first end margin 210 of the main body 202 in a manner such that the first end margin of the main body can pivot about an axis that is generally parallel to the wire mount 218 and perpendicular to the longitudinal direction of the main body 202. The second mount 206 of the lumbar support device 200 preferably comprises a pair of brackets 222 that connect opposite side portions 224 of the second end margin 212 of the main body 202 to the sides 216 or top of the seat frame 214. Each of such brackets 222 are similar to the bracket 110 of the alternative embodiment of the lumbar support device 10 shown in FIG. 4 and discussed above and, similarly, each comprises a hook 226. Like the bracket 110 of the alternative embodiment of the lumbar support device 10 shown in FIG. 4, the hook 226 holds the respective side portion 224 of the second end margin 212 of the main body 202 in a manner that allows the second end margin to slide a given distance toward the first end margin 210 relative to the bracket and thereafter only permits rotation.

The main body 202 is preferably formed from a single sinuate wire 228 that preferably extends longitudinally from the first end margin 210 of the main body, to the second end margin 212 of the main body, and back to the first end margin in a manner such that two undulating springs 230 are formed traversing the intermediate portion 213 of the main body 202. The two undulating springs 230 can be generally referred to as two intermediate portions of the main body 202. A lever arm 232 is formed by the sinuate wire 228 at the second end margin 212 of the main body 202 as the wire loops back toward the first end margin 210. As best shown in FIG. 9, the lever arm 232 at the second end margin 212 of the main body 202 cantilevers from the main body 202 and creates a moment-arm about an axis that extends through each of the hooks 226 of the brackets 222 of the second mount 206. In a similar manner, the opposite ends 234 of the sinuate wire 228 turn abruptly at the first end margin 210 of the main body 202 and together form a pair of lever arms 236 that cantilever therefrom, such that each of said lever arms forms a moment-arm with respect to the center axis of the wire mount 218 of the first mount 204. The lever arms 236 of the first end margin 210 are preferably joined by and held spaced apart by the connector 220 of the first mount 204. As shown, the lever arms 236 at the first end margin 210 is preferably dimensioned slightly longer than the lever arm 232 at second end margin 212 of the of the main body 202.

An important aspect of the main body 202 of the fourth embodiment of the lumbar support device 200 lies in the configuration of the undulating springs 230. Unlike the embodiments discussed above, the sinuous pattern of the trajectory of each of the undulating springs 230 of the fourth embodiment is not uniform as each such spring 230 longitudinally traverses the intermediate portion 213 of the main body 202. In particular, the intermediate portion 213 of the main body 202 is theoretically divisible a some point along its longitudinal trajectory into first 238 and second 240 longitudinally extending sub-portions that have different bending stiffnesses. The difference in bending stiffnesses is preferably achieved by changing the sinuous pattern of each undulating spring 230 as it longitudinally traverses the intermediate portion 213 of the main body. In particular sinuous pattern of each of the undulating springs 230 within the first sub-portion 238 preferably has a greater average frequency and a greater average amplitude than it averages throughout the second sub-portion 240. As a direct result of such, it should be appreciated by one skilled in the art that the first sub-portion 238 of the intermediate portion 213 has a bending stiffness that is substantially less than that of the second sub-portion 240, thereby making the first sub-portion more easy to flex than the second sub-portion. This difference in flexibility impacts the shape of the longitudinal trajectory of the main body 202 when subjected to bending stresses and is utilized, as discussed below, to bow the main body in a non-symmetrical, preferred manner.

The actuator assembly 208 of the lumbar support device 200 of the fourth embodiment is preferably similar to that of the preferred embodiment shown in FIGS. 1-3 and the specific type and arrangement of the actuator assembly is not of particular importance to this embodiment. It should be appreciated that the actuator assembly 208 preferably comprises a bowden cable 242 that is configured and adapted to selectively generate a tractive force between objects. As shown in most clearly in FIG. 9, the actuator assembly 208 is operatively connected to the lever arms 232, 236 at each of the end margins 210, 212 of the main body 202 where, when activated, the actuator assembly will act to force the levers toward each other.

In operation, lumbar support device of the fourth embodiment generally functions in a manner similar to the lumbar support device 10 shown in FIGS. 4 and 5. In a first position, as shown in solid lines in FIG. 9, the longitudinal trajectory of the intermediate portion 213 of the main member 202 extends substantially strait. Additionally, when in the first position, the second end margin 212 of the main body 202 is free to slide a given distance in a direction toward the first end margin 210 relative to the brackets 222 of the second mount 206 due to the configuration of the hooks 226 as described above in reference to FIGS. 4 and 5. When desired, the actuator assembly 208 can be activated to generate a tractive force between the lever arms 232, 236 of the end margins 210, 212 of the main body 202. Similar to the other embodiments discussed above, the tractive force on lever arms 232, 236 induces a bending moment in each of the end margins 210, 212 which, in turn, causes longitudinal trajectory of the intermediate portion 213 to bend to a second position in which it bows perpendicular outward relative to its lateral width, as shown in dashed lines in FIG. 9.

Unlike the previously described embodiments of lumber support devices, the main body 202 of the lumbar support device 200 of the fourth embodiment bows in a non-symmetric manner. This non-symmetric bowing is due, at least in part, to the configuration of the intermediate portion 213, as described above, that results in the bending stiffness of its first sub-portion being substantially less than that of the second sub-portion 240. It should be appreciated that, as a result of such stiffnesses, for any given induced bending moment, the longitudinal trajectory of the intermediate portion 213 of the main body 202 throughout its first sub-portion 238 will increase in a greater average curvature than that of the second sub-potion 240. Thus, the longitudinal trajectory of the first sub-portion 213 will therefore tend to bulge further outwardly throughout the first sub-portion 238 as compared to the second sub-portion 240. Although the bending moment induced throughout the main body 202 via the actuator assembly 208 is not necessarily constant, it should also be appreciated that the differences in bending stiffness between the first and second sub-portions 238, 240 at least partially contributes to any such bulging. It should also be appreciated that the main body 202 is preferably oriented in a manner such that its first longitudinal half 238 is positioned below its second half 240 with respect to the seat frame 214 such that the first half of the main body is positioned adjacent a person's lumber region when such a person rest against the seat. Thus, the uneven bulging or bowing of the first sub-portion 238 of the intermediate portion 213 of the main body 202 compared to the second sub-portion 240 is desirable for providing additional lumbar support when needed.

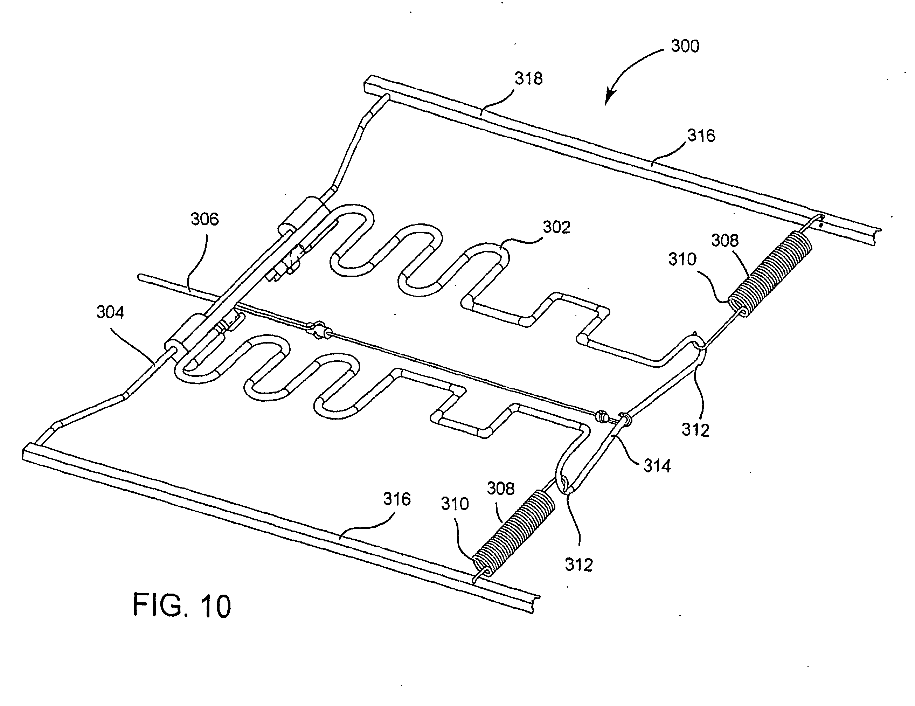

The lumbar support device 300 of the fifth embodiment shown in FIG. 10 is substantially identical to the lumbar support device 200 of the fourth embodiment and utilizes an identical main body 302, first mount 304, and actuator assembly 306. However, second mount 308 of the lumber support device 300 of the fifth embodiment comprises a pair of coil tension springs 310 to operatively connect the opposite side portions 312 of the second end margin 314 of the main body 302 to the sides 316 of the seat frame 318 in which the device is placed.

In operation, the coil tension springs 310 operate in manner similar to the brackets 222 of the lumbar support device 200 of the fourth embodiment in that they allow the second end margin 314 of the main body 302 to translate toward the first end margin 320 of the main body as main body is bowed via the actuator assembly 306. However, unlike the brackets 222 of the lumbar support device 200 of the fourth embodiment, the coil tension springs 310 allow such motion by resiliently stretching and therefore no relative sliding between the coil tension springs and the main body 302 occurs. Thus, the lumbar support device 302 of the fifth embodiment has less tendency to wear or bind during use and is simplified as compared to the lumbar support device 202 of the fourth embodiment discussed above.

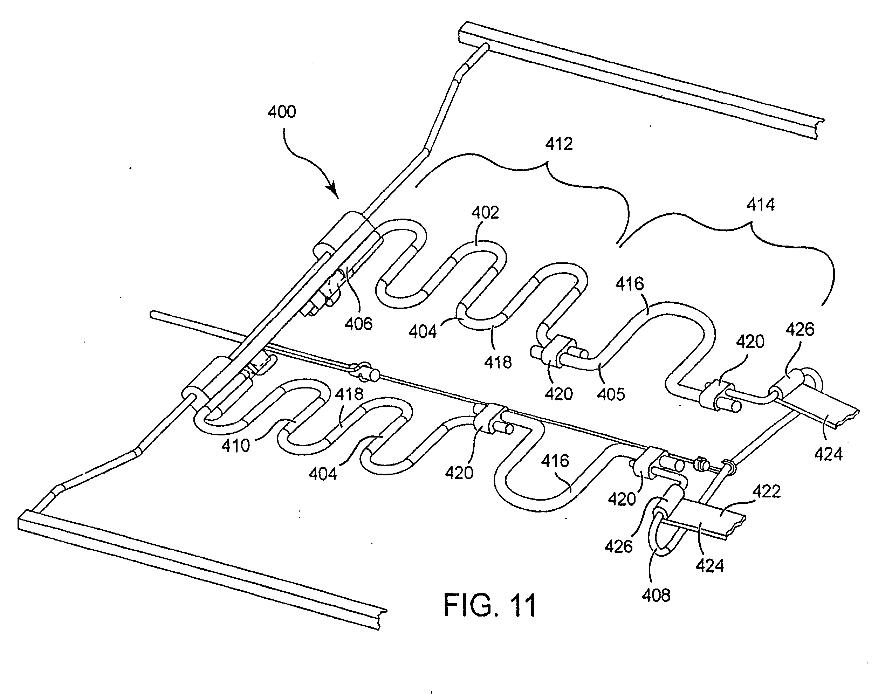

FIG. 11 illustrates yet another embodiment of a lumbar support device 400. This sixth embodiment of a lumber support device 400 is configured and adapted to achieve the same results as the fourth and fifth embodiments discussed above, but does so using a differently configure main body 402. Like the main bodies 202, 302 of the fourth and fifth embodiments, the main body 402 of the lumber support device 400 of the sixth embodiment comprises a pair of undulating springs 404 that longitudinally traverse the intermediate portion 405 of the main body between first and second end margins 406, 408 of the main body. However, unlike the lumbar support devices 200, 300 of the fourth and fifth embodiments, the wire 410 forming each of the undulating springs 404 changes gauges as it extends longitudinally in a manner such that the average diameter of the wire throughout a first sub-portion 412 of the intermediate portion 405 is less than average diameter of the wire throughout a second sub-portion 414 of the intermediate portion. As shown in FIG.11, this can be done by crimping a lager diameter wire 416 section to a smaller diameter wire section 418 via annular bands 420. Alternatively, different gauge wire sections can be butt-welded end to end to form a continuous wire of varying gauge (not shown), or other suitable techniques know in the art can be utilized to achieve the same result, such as initially forming the wire of different gauges along its longitudinal trajectory or by using wire sections having different material properties.

As a result of comprising more than one gauge of wire as discussed above, the first sub-portion 412 of the intermediate portion 405 of the main body 402 has significantly less bending stiffness than that of the second sub-portion 414, without necessarily having a different sinuate pattern. Thus, it should be appreciated that the first sub-portion 412 of the intermediate portion 405 of the main body 402 will have a tendency to increase in curvature more than the second sub-portion 414 as the main body is subjected to bending moments.

Another difference between the lumbar support device 400 of the sixth embodiment as compared to the lumbar support devices 200, 300 of the fourth and fifth embodiments is the second mount 422. The second mount 422 of the lumbar support device 400 of the sixth embodiment comprises a pair of brackets 424 having closed loop ends 426 that allow only pivotal movement between the second end margin 408 of the main body 402 and such brackets, similar to the brackets shown in FIGS. 1-3 and discussed above.

Aside from the differences discussed above, the lumbar support device 400 of the sixth embodiment is identical to the lumbar support devices 200, 300 of the fourth and fifth embodiments and it should therefore be appreciated that the main body 402 of the lumbar support device of this sixth embodiment operates much like the main body 202, 302 of the fourth and fifth embodiments and achieves a similarly desirable uneven flexing when actuated.

As various modifications could be made in the constructions and methods herein described and illustrated without departing from the scope of the invention, it is intended that all matter contained in the foregoing description or shown in the accompanying drawings shall be interpreted as illustrative rather than limiting. For example, while a sinusoidal spring and a leaf spring are particularly illustrated for the present invention, it will be evident to those skilled in the art that other types of integrally formed springs or combination of springs, such as a composite spring made with a leaf spring and a sinusoidal spring, or a combination using a coil spring, may be interchanged with the illustrated springs. Thus, the breadth and scope of the present invention should not be limited by any of the above-described exemplary embodiments, but should be defined only in accordance with the following claims appended hereto and their equivalents.

## Claims

1. A lumbar support device, comprising:
a first mount (18; 110; 124; 204; 304) having a first fulcrum;
a second mount (20; 114; 126; 206; 308; 422) located distally from the first mount;
a spring assembly (22; 102; 122; 202; 302; 402) extending along a longitudinal trajectory and having a first cantilevered end, a second end and a center section, the first cantilevered end rotatably connected to and cantilevered about the first mount (18; 110; 124; 204; 304), the second end connected to the second mount (20; 114; 126; 206; 308; 422), and the center section extending between the first mount and the second mount, wherein the first cantilevered end defines a first lever (40; 104; 120; 236) extending from the first fulcrum to a first distal end such that the first fulcrum is located between the first distal end and the center section;
an actuator assembly (30; 208; 306) operatively connected to the first distal end of the first lever (40; 104; 120; 236), wherein the actuator assembly (30; 208; 306) moves the first distal end and the first fulcrum stops the first lever (40; 104; 120; 236) from sliding and forces the first lever (40; 104; 120; 236) to rotate,
**characterized in that**
the first mount (18; 110; 124; 204; 304) is attached to a first side of a seat frame (12; 214; 318) and the second mount is attached to a second side of the seat frame (12; 214; 318),
the actuator assembly (30; 208; 306) being configured and adapted to selectively induce an increasing bending moment in the first cantilevered end of the spring assembly (22; 102; 122; 202; 302; 402) such that the longitudinal trajectory of the spring assembly increases in curvature in response to the increasing bending moment.

2. A lumbar support device according to claim 1 wherein the center section is integrally formed with the first cantilevered end and the second end.

3. A lumbar support device according to claim 1 wherein the center section is fixedly attached to the first cantilevered end and the second end.

4. A lumbar support device according to any one of claims 1 to 3 wherein the spring assembly (22; 102; 122i; 202; 302; 402) comprises at least one sinusoidal spring or a leaf spring.

5. A lumbar support device according to claim 1 wherein the spring assembly (22; 102; 202; 302; 402) essentially consists of wire and extends between longitudinally opposite first and second end margins of the spring assembly.

6. A lumbar support device according to claim 5, further comprising:
a connector (220) pivotally connected to the first mount (204; 304) and to the first end margin of the spring assembly (202; 302; 402) in a manner such that the first end margin of the spring assembly (202; 302; 402) can pivot about an axis that is generally parallel to the first mount (204; 304).

7. A lumbar support device according to claim 5 or 6 wherein the wire (228; 410) comprises at least one continuous wire that extends from the first end margin to the second end margin, the at least one continuous wire being formed in a manner such that that it has a sinuate shape as the continuous wire extends along the longitudinal trajectory.

8. A lumbar support device according to claim 7 wherein the at least one continuous wire has a generally constant diameter.

9. A lumbar support device in according to claim 7 wherein the wire (228) consists of the one continuous wire.

10. A lumbar support device according to any one of claims 6 to 9 wherein the first lever (236) is formed by the continuous wire.

11. A lumbar support device according to any one of claims 6 to 10 wherein the continuous wire further comprises at least two intermediate portions (213) between the first end margin end and the second end margin.

12. A lumbar support device according to any one of claims 5 to 11 wherein the wire (228; 410) forms a second lever (232) at the second end margin, the second lever (232) extending in a cantilever manner relative to the second mount (206; 308; 422) and the actuator assembly being configured and adapted to induce a bending moment in the second end margin of the main body via the second lever.

13. A lumbar support device according to claims 11 and 12 wherein the second lever (232) is formed by the continuous wire between the two intermediate portions (213).

14. A lumbar support device according to any one of claims 5 to 13 wherein the center section of the spring assembly (202; 302; 402) further comprises an intermediate portion (213), the intermediate portion further comprising a first sub-portion (238; 412) and a second sub-portion (240; 414), wherein the first sub-portion (238; 412) has an average bending stiffness that is less than an average bending stiffness of the second sub-portion (240; 412).

15. A lumbar support device according to claim 14 wherein the longitudinal trajectory along each of the first and second sub-portions (238, 240; 412, 414) has a curvature change when the longitudinal trajectory is bent between first and second positions via the actuator assembly (208; 306), the curvature change of the longitudinal trajectory along the first sub-portion (238; 412) being greater than the curvature change of the longitudinal trajectory along the second sub-portion (240; 414) according to the difference in the average bending stiffness between the first sub-portion (238; 412) and the second sub-portion (240; 414).

16. A lumbar support device according to claim 14 or 15 wherein the wire (228) comprises at least one continuous wire that extends from the first end margin to the second end margin, the at least one continuous wire being formed in a manner such that that it has a sinuate shape as the continuous wire extends along the longitudinal trajectory, the sinuate shape having a greater average frequency and a greater average amplitude throughout the first sub-portion (238) than the sinuate shape in the second sub-portion (240).

17. A lumbar support device according to claim 14 or 15, wherein the wire (410) comprises a larger diameter wire in the first sub-portion (412) and a smaller diameter wire in the second sub-portion (414).

18. A lumbar support device according to any one of claims 14 to 17 wherein the first sub-portion (238; 412) of the spring assembly (202; 302; 402) separates the first end margin from the second sub-portion (240; 414) as the spring assembly extends along the longitudinal trajectory.

19. A lumbar support device according to any one of claims 1 to 3 wherein the first fulcrum of the first mount (18; 110) is a hook (112) limiting the first lever (40; 104) from sliding or a loop preventing the first lever (40; 104) from sliding.

20. A lumbar support device according to any one of claims 1 to 3 wherein the first mount (18; 110; 124; 204; 304) further comprises a coil spring.

21. A lumbar support device according to any one of claims 1 to 3 wherein the actuator assembly (30; 208; 306) tractively moves the first distal end or pulsively moves the first distal end.

22. A lumbar support device according to any one of claims 1 to 3 wherein the second mount (20; 126; 206; 308; 422) further comprises a second fulcrum and the second end is rotatably connected to and cantilevered about the second mount (20; 126; 206; 308; 422), the second end defining a second lever (42; 232) extending from the second fulcrum to a second distal end, and wherein the actuator assembly (30; 208; 306) operatively connects the first distal end with the second distal end.

23. A lumbar support device according to claim 21 wherein the actuator assembly (30) further comprises a bowden cable assembly (46) and an actuator (48), the actuator (48) operatively engaging the bowden cable assembly (48) and the bowden cable assembly (48) tractively linking the first distal end with the second distal end.

24. A lumbar support device according to claim 22 or 23 wherein the spring assembly (22; 102) is further comprised of a sinusoidal spring (58, 60) having a first loop (66), a last loop (68) and a plurality of loops (70), the first loop (66) defining the first cantilevered end, the last loop (68) defining the second cantilevered end, and the plurality of loops (70) defining the center section, wherein the plurality of loops (70) is further comprised of a second loop (72) and a second-to-last loop (74), the second loop (72) being integrally formed with the first loop (66) and the second-to-last loop (74) being integrally formed with the last loop (68).

25. A lumbar support device according to any one of claims 1 to 3 wherein the spring assembly (22) comprises a first spring (58) and a second spring (60) and a pair of connectors (62, 64), the pair of connectors (62, 64) attaching the first spring (58) with the second spring (60).

26. A lumbar support device according to any one of the claims 1 to 3 wherein the center section of the spring assembly (22) is further comprised of a recessed portion (44).

27. A lumbar support device according to any one of claims 5 to 18 wherein the second mount (126; 206; 308) is configured such that the second end of the spring assembly (122; 202; 302) is permitted to translate relative to the second mount (124; 206; 308) in a direction toward the first mount (124; 204; 304) when the spring assembly (122; 202; 302) increases in curvature or the longitudinal trajectory is initially bent via the actuator.

28. A lumbar support device in according to any one of claims 5 to 18 wherein the second mount (308) further comprises a pair of coil springs (310).

## Patentansprüche

1. Lendenstützvorrichtung, umfassend:
eine erste Halterung (18; 110; 124; 204; 304), welche einen ersten Stützpunkt besitzt;
eine zweite Halterung (20; 114; 126; 206; 308; 422), welche von der ersten Halterung beabstandet angeordnet ist;
eine Federanordnung (22; 102; 122; 202; 302; 402), welche entlang einer Längstrajektorie verläuft und ein erstes freitragendes Ende, ein zweites Ende und einen mittleren Abschnitt besitzt, wobei das erste freitragendes Ende drehbar mit der ersten Halterung (18; 110; 124; 204; 304) verbunden und um die erste Halterung (18; 110; 124; 204; 304) freitragend angeordnet ist, wobei das zweite Ende mit der zweiten Halterung (20; 114; 126; 206; 308; 422) verbunden ist und der mittlere Abschnitt zwischen der ersten Halterung und der zweiten Halterung verläuft, wobei das erste freitragende Ende einen ersten Hebel (40; 104; 120; 236) definiert, welcher von dem ersten Stützpunkt zu einem ersten distalen Ende verläuft, sodass der erste Stützpunkt zwischen dem ersten distalen Ende und dem mittleren Abschnitt angeordnet ist;
eine Betätigungsanordnung (30; 208; 306), welche mit dem ersten distalen Ende des ersten Hebels (40; 104; 120; 236) wirksam verbunden ist, wobei die Betätigungsanordnung (30; 208; 306) das erste distale Ende bewegt und der erste Stützpunkt den ersten Hebel (40; 104; 120; 236) vom Rutschen abhält und
den ersten Hebel (40; 104; 120; 236) zum Drehen zwingt,
**dadurch gekennzeichnet,**
**dass** die erste Halterung (18; 110; 124; 204; 304) an einer ersten Seite eines Sitzrahmens (12; 214; 318) angebracht ist und die zweite Halterung an einer zweiten Seite des Sitzrahmens (12; 214; 318) angebracht ist,
die Betätigungsanordnung (30; 208; 306) ausgestaltet und angepasst ist, um wahlweise ein zunehmendes Biegemoment an dem ersten freitragenden Ende der Federanordnung (22; 102; 122; 202; 302; 402) einzubringen, sodass die Längstrajektorie der Federanordnung hinsichtlich ihrer Krümmung als Reaktion auf das zunehmende Biegemoment zunimmt.

2. Lendenstützvorrichtung nach Anspruch 1, wobei der mittlere Abschnitt mit dem ersten freitragenden Ende und dem zweiten Ende als Einheit ausgebildet ist.

3. Lendenstützvorrichtung nach Anspruch 1, wobei der mittlere Abschnitt fest an dem ersten freitragenden Ende und dem zweiten Ende angebracht ist.

4. Lendenstützvorrichtung nach einem der Ansprüche 1-3, wobei die Federanordnung (22; 102; 122i; 202; 302; 402) mindestens eine sinusförmig verlaufende Feder oder eine Blattfeder umfasst.

5. Lendenstützvorrichtung nach Anspruch 1, wobei die Federanordnung (22; 102; 202; 302; 402) hauptsächlich aus Draht besteht und zwischen gegenüberliegenden ersten und zweiten Endrändern der Federanordnung in Längsrichtung verläuft.

6. Lendenstützvorrichtung nach Anspruch 5, weiter umfassend:
einen Verbinder (220), welcher mit der ersten Halterung (204; 304) und dem ersten Endrand der Federanordnung (202; 302; 402) in der Weise schwenkbar verbunden ist, sodass der erste Endrahmen der Federanordnung (202; 302; 402) um eine Achse schwenken kann, welche im Allgemeinen parallel zu der ersten Halterung (204; 304) ist.

7. Lendenstützvorrichtung nach Anspruch 5 oder 6, wobei der Draht (228; 410) mindestens einen durchgehenden Draht umfasst, welcher von dem ersten Endrand zu dem zweiten Endrand verläuft, wobei der mindestens eine durchgehende Draht in der Weise ausgebildet ist, dass er eine Sinusform besitzt während er entlang der Längstrajektorie verläuft.

8. Lendenstützvorrichtung nach Anspruch 7, wobei der mindestens eine durchgehende Draht einen im Allgemeinen konstanten Durchmesser besitzt.

9. Lendenstützvorrichtung nach Anspruch 7, wobei der Draht (228) aus dem einen durchgehenden Draht besteht.

10. Lendenstützvorrichtung nach einem der Ansprüche 6-9, wobei der erste Hebel (236) durch den durchgehenden Draht gebildet ist.

11. Lendenstützvorrichtung nach einem der Ansprüche 6-10, wobei der durchgehende Draht weiterhin mindestens zwei Zwischenabschnitte (213) zwischen dem ersten Endrand und dem zweiten Endrand umfasst.

12. Lendenstützvorrichtung nach einem der Ansprüche 5-11, wobei der Draht (228; 410) einen zweiten Hebel (232) an dem zweiten Endrand ausbildet, wobei der zweite Hebel (232) in einer freitragenden Weise bezüglich der zweiten Halterung (206; 308; 422) verläuft und die Betätigungsanordnung ausgebildet und angepasst ist, um ein Biegemoment an dem zweiten Endrand des Hauptkörpers über den zweiten Hebel einzubringen.

13. Lendenstützvorrichtung nach Anspruch 11 und 12, wobei der zweite Hebel (232) durch den durchgehenden Draht zwischen den zwei Zwischenabschnitten (213) gebildet ist.

14. Lendenstützvorrichtung nach einem der Ansprüche 5-13, wobei der mittlere Abschnitt der Federanordnung (202; 302; 402) weiter einen Zwischenabschnitt (213) umfasst, wobei der Zwischenabschnitt weiterhin einen ersten Unterabschnitt (238; 412) und einen zweiten Unterabschnitt (240; 414) umfasst, wobei der erste Unterabschnitt (238; 412) eine durchschnittliche Biegesteifheit besitzt, welche geringer ist als eine durchschnittliche Biegesteifheit des zweiten Unterabschnitts (240; 412).

15. Lendenstützvorrichtung nach Anspruch 14, wobei die Längstrajektorie entlang jeder der ersten und zweiten Unterabschnitte (238, 240; 412, 414) einen Krümmungsveränderung besitzt, wenn die Längstrajektorie zwischen einer ersten und zweiten Stellung durch die Betätigungsanordnung (208; 306) gekrümmt wird, wobei die Krümmungsänderung der Längstrajektorie entlang des ersten Unterabschnitts (238; 412) größer ist als die Krümmungsänderung der Längstrajektorie entlang des zweiten Unterabschnitts (240; 414) gemäß dem Unterschied der durchschnittlichen Biegesteifheit zwischen dem ersten Unterabschnitt (238; 414) und dem zweiten Unterabschnitt (240; 414).

16. Lendenstützvorrichtung nach Anspruch 14 oder 15, wobei der Draht (228) mindestens einen durchgehenden Draht umfasst, welcher von dem ersten Endrand zu dem zweiten Endrand verläuft, wobei der mindestens eine durchgehende Draht in einer Weise ausgebildet ist, sodass er eine Sinusform besitzt während der durchgehende Draht entlang der Längstrajektorie verläuft, wobei die Sinusform eine größere Durchschnittsfrequenz und eine größere Durchschnittsamplitude über dem gesamten ersten Unterabschnitt (238) als die Sinusform in dem zweiten Unterabschnitt (240) besitzt.

17. Lendenstützvorrichtung nach Anspruch 14 oder 15, wobei der Draht (410) einen größeren Drahtdurchmesser in dem ersten Unterabschnitt (412) und einen kleineren Drahtdurchmesser in dem zweiten Unterabschnitt (414) umfasst.

18. Lendenstützvorrichtung nach einem der Ansprüche 14-17, wobei der erste Unterabschnitt (238; 412) der Federanordnung (202; 302; 402) den ersten Endrand von dem zweiten Unterabschnitt (240; 414) trennt während die Federanordnung entlang der Längstrajektorie verläuft.

19. Lendenstützvorrichtung nach einem der Ansprüche 1-3, wobei der erste Stützpunkt der ersten Halterung (18; 110) ein Haken (112) ist, welcher ein Verrutschen der ersten Hebels (40; 104) begrenzt, oder eine Schlaufe, welche ein Verrutschen des ersten Hebels (40; 104) verhindert.

20. Lendenstützvorrichtung nach einem der Ansprüche 1-3, wobei die erste Halterung (18; 110; 124; 204; 304) weiter eine Spiralfeder umfasst.

21. Lendenstützvorrichtung nach einem der Ansprüche 1-3, wobei die Betätigungsanordnung (30; 208; 306) das erste distale Ende zieht oder das erste distale Ende pulsierend bewegt.

22. Lendenstützvorrichtung nach einem der Ansprüche 1-3, wobei die zweite Halterung (20; 126; 206; 308; 422) weiter einen zweiten Stützpunkt umfasst und das zweite Ende drehbar mit der zweiten Halterung (20; 126; 206; 308; 422) verbunden und freitragend um die zweite Halterung (20; 126; 206; 308; 422) angeordnet ist, wobei das zweite Ende einen zweiten Hebel (42; 232) definiert, welcher von dem zweiten Stützpunkt zu einem zweiten distalen Ende verläuft, und wobei die Betätigungsanordnung (30; 208; 306) wirksam das erste distale Ende mit dem zweiten distalen Ende verbindet.

23. Lendenstützvorrichtung nach Anspruch 21, wobei die Betätigungsanordnung (30) weiterhin eine Bowdenzuganordnung (46) und einen Betätiger (48) umfasst, wobei der Betätiger (48) sich wirksam mit der Bowdenzuganordnung (48) in Eingriff befindet und die Bowdenzuganordnung (48) das erste distale Ende mit dem zweiten distalen Ende ziehend verbindet.

24. Lendenstützvorrichtung nach Anspruch 22 oder 23, wobei die Federanordnung (22; 102) weiterhin eine sinusförmige Feder (58, 60) umfasst, welche eine erste Schleife (66), eine letzte Schleife (68) und eine Vielzahl von Schleifen (70) besitzt, wobei die erste Schleife (66) das erste freitragende Ende definiert, die letzte Schleife (68) das zweite freitragende Ende definiert und die Vielzahl von Schleifen (70) den mittleren Abschnitt definieren, wobei die Vielzahl von Schleifen (70) weiterhin eine zweite Schleife (72) und eine vorletzte Schleife (74) umfasst, wobei die zweite Schleife (72) mit der ersten Schleife (66) als Einheit ausgebildet ist und die vorletzte Schleife (74) mit der letzten Schleife (68) als Einheit ausgebildet ist.

25. Lendenstützvorrichtung nach einem der Ansprüche 1-3, wobei die Federanordnung (22) eine erste Feder (58) und eine zweite Feder (60) und ein Paar von Verbindern (62, 64) umfasst, wobei das Paar von Verbindern (62, 64) die erste Feder (58) an der zweiten Feder (60) anbringt.

26. Lendenstützvorrichtung nach einem der Ansprüche 1-3, wobei der mittlere Abschnitt der Federanordnung (22) weiterhin einen vertieften Abschnitt (44) umfasst.

27. Lendenstützvorrichtung nach einem der Ansprüche 5-18, wobei die zweite Halterung (126; 206; 308) derart ausgestaltet ist, dass das zweite Ende der zweiten Federanordnung (122; 202; 302) sich bezüglich der ersten Halterung (124; 206; 308) in Richtung der ersten Halterung (124; 204; 304) verlagern kann, wenn die Krümmung der Federanordnung (122; 202; 302) zunimmt oder die Längstrajektorie anfangs durch den Betätiger gekrümmt wird.

28. Lendenstützvorrichtung nach einem der Ansprüche 5-18, wobei die zweite Halterung (308) des Weiteren ein Paar von Spiralfedern (310) umfasst.

## Revendications

1. Dispositif de support de lombaire, comprenant :
une première monture (18, 110, 124, 204, 304) ayant un premier pivot ;
une deuxième monture (20, 114, 126, 206, 308, 422) disposée de façon distale de la première monture ;
un ensemble de ressort (22, 102, 122, 202, 302, 402) s'étendant le long d'une trajectoire longitudinale et ayant une première extrémité en porte-à-faux, une deuxième extrémité et une section centrale, la première extrémité en porte-à-faux étant reliée de façon rotative et en porte-à-faux autour de la première monture (18, 110, 124, 204, 304), la deuxième extrémité étant reliée à la deuxième monture (20, 114, 126, 206, 308, 422) et la section centrale s'étendant entre la première monture et la deuxième monture, dans lequel la première extrémité en porte-à-faux définit un premier levier (40, 104, 120, 236) s'étendant à partir du premier pivot à une première extrémité distale, de sorte que le premier pivot est situé entre la première extrémité distale et la section centrale ;
un ensemble d'actionneur (30, 208, 306) relié de façon opérationnelle à la première extrémité distale du premier levier (40, 104, 120, 236), dans lequel l'ensemble d'actionneur (30, 208, 306) déplace la première extrémité distale et le premier pivot arrête le premier levier (40, 104, 120, 236) du coulissement et oblige le premier levier (40, 104, 120, 236) à tourner,
**caractérisé en ce que** :
la première monture (18, 110, 124, 204, 304) est fixée à un premier côté d'un cadre de siège (12, 214, 318) et la deuxième monture est fixée à un deuxième côté du cadre de siège (12, 214, 318),
l'ensemble d'actionneur (30, 208, 306) étant configuré et agencé pour induire sélectivement un moment de flexion augmentant dans la première extrémité en porte-à-faux de l'ensemble de ressort (22, 102, 122, 202, 302, 402) de sorte que la trajectoire longitudinale de l'ensemble de ressort augmente en courbure en réponse au moment de flexion augmentant.

2. Dispositif de support de lombaire selon la revendication 1, dans lequel la section centrale est formée de façon solidaire avec la première extrémité en porte-à-faux et la deuxième extrémité.

3. Dispositif de support de lombaire selon la revendication 1, dans lequel la section centrale est fixée fixement à la première extrémité en porte-à-faux et la deuxième extrémité.

4. Dispositif de support de lombaire selon l'une quelconque des revendications 1 à 3, dans lequel l'ensemble de ressort (22, 102, 122i, 202, 302, 402) comprend au moins un ressort sinusoïdal ou un ressort lame.

5. Dispositif de support de lombaire selon la revendication 1, dans lequel l'ensemble de ressort (22, 102, 202, 302, 402) se compose essentiellement de fil et s'étend entre les première et deuxième lisières d'extrémité longitudinalement opposées de l'ensemble de ressort.

6. Dispositif de support de lombaire selon la revendication 5, comprenant, en outre :
un connecteur (220) relié de façon pivotante à la première monture (204, 304) et à la première lisière d'extrémité de l'ensemble de ressort (202, 302, 402) de façon que la première lisière d'extrémité de l'ensemble de ressort (202, 302, 402) puisse pivoter autour d'un axe qui est généralement parallèle à la première monture (204, 304).

7. Dispositif de support de lombaire selon la revendication 5 ou 6, dans lequel le fil (228, 410) comprend au moins un fil continu qui s'étend à partir de la première lisière d'extrémité à la deuxième lisière d'extrémité, le au moins un fil continu étant formé de sorte qu'il présente une forme sinueuse lorsque le fil continu s'étend le long de la trajectoire longitudinale.

8. Dispositif de support de lombaire selon la revendication 7, dans lequel le au moins un fil continu présente un diamètre généralement constant.

9. Dispositif de support de lombaire selon la revendication 7, dans lequel le fil (228) se compose du un fil continu.

10. Dispositif de support de lombaire selon l'une quelconque des revendications 6 à 9, dans lequel le premier levier (236) est formé par le fil continu.

11. Dispositif de support de lombaire selon l'une quelconque des revendications 6 à 10, dans lequel le fil continu comprend, en outre, au moins deux parties intermédiaires (213) entre la première extrémité de lisière d'extrémité et la deuxième lisière d'extrémité.

12. Dispositif de support de lombaire selon l'une quelconque des revendications 5 à 11, dans lequel le fil (228, 410) forme un deuxième levier (232) au niveau de la deuxième lisière d'extrémité, le deuxième levier (232) s'étendant en porte-à-faux par rapport à la deuxième monture (206, 308, 422) et l'ensemble d'actionneur étant configuré et agencé pour induire un moment de flexion dans la deuxième lisière d'extrémité du corps principal par l'intermédiaire du deuxième levier.

13. Dispositif de support de lombaire selon les revendications 11 et 12, dans lequel le deuxième levier (232) est formé par le fil continu entre les deux parties intermédiaires (213).

14. Dispositif de support de lombaire selon l'une quelconque des revendications 5 à 13, dans lequel la section centrale de l'ensemble de ressort (202, 302, 402) comprend, en outre, une partie intermédiaire (213), la partie intermédiaire comprenant, en outre, une première sous-partie (238, 412) et une deuxième sous-partie (240, 414), dans lequel la première sous-partie (238, 412) présente une rigidité de flexion moyenne qui est inférieure à la rigidité de flexion moyenne de la deuxième sous-partie (240, 412).

15. Dispositif de support de lombaire selon la revendication 14, dans lequel la trajectoire longitudinale le long de chacune des première et deuxième sous-parties (238, 240, 412, 414) présente une modification de courbure lorsque la trajectoire longitudinale est courbée entre les première et deuxième positions par l'intermédiaire de l'ensemble d'actionneur (208, 306), la modification de courbure de la trajectoire longitudinale le long de la première sous-partie (238, 412) étant supérieure à la modification de courbure de la trajectoire longitudinale le long de la deuxième sous-partie (240, 414) conformément à la différence dans la rigidité de flexion moyenne entre la première sous-partie (238, 412) et la deuxième sous-partie (240, 414).

16. Dispositif de support de lombaire selon la revendication 14 ou 15, dans lequel le fil (228) comprend au moins un fil continu qui s'étend de la première lisière d'extrémité à la deuxième lisière d'extrémité, le au moins un fil continu étant formé de façon telle qu'il présente une forme sinueuse lorsque le fil continu s'étend le long de la trajectoire longitudinale, la forme sinueuse ayant une fréquence moyenne supérieure et une amplitude moyenne supérieure au travers de la première sous-partie (238) que la forme sinueuse dans la deuxième sous-partie (240).

17. Dispositif de support de lombaire selon la revendication 14 ou 15, dans lequel le fil (410) comprend un diamètre de fil plus grand dans la première sous-partie (412) et un diamètre de fil plus petit dans la deuxième sous-partie (414).

18. Dispositif de support de lombaire selon l'une quelconque des revendications 14 à 17, dans lequel la première sous-partie (238, 412) de l'ensemble de ressort (202, 302, 402) sépare la première lisière d'extrémité de la deuxième sous-partie (240, 414) lorsque l'ensemble de ressort s'étend le long de la trajectoire longitudinale.

19. Dispositif de support de lombaire selon l'une quelconque des revendications 1 à 3, dans lequel le premier pivot de la première monture (18, 118) est un crochet (112) limitant le premier levier (40, 104) d'un coulissement ou une boucle empêchant le premier levier (40, 104) de glisser.

20. Dispositif de support de lombaire selon l'une quelconque des revendications 1 à 3, dans lequel la première monture (18, 110, 124, 204, 304) comprend, en outre, un ressort hélicoïdal.

21. Dispositif de support de lombaire selon l'une quelconque des revendications 1 à 3, dans lequel l'ensemble d'actionneur (30, 208, 306) déplace à traction la première extrémité distale et déplace à pulsion la première extrémité distale.

22. Dispositif de support de lombaire selon l'une quelconque des revendications 1 à 3, dans lequel la deuxième monture (20, 126, 206, 308, 422) comprend, en outre, un deuxième pivot et la deuxième extrémité est reliée à rotation et en porte-à-faux autour de la deuxième monture (20, 126, 206, 308, 422), la deuxième extrémité définissant un deuxième levier (42, 232) s'étendant à partir du deuxième pivot à une deuxième extrémité distale et dans lequel l'ensemble d'actionneur (30, 208, 306) relie de façon opérationnelle la première extrémité distale à la deuxième extrémité distale.

23. Dispositif de support de lombaire selon la revendication 21, dans lequel l'ensemble d'actionneur (30) comprend, en outre, un ensemble de câble Bowden (46) et un actionneur (48), l'actionneur (48) mettant en prise de façon opérationnelle l'ensemble de câble Bowden (48) et l'ensemble de câble Bowden (48) reliant à traction la première extrémité distale à la deuxième extrémité distale.

24. Dispositif de support de lombaire selon la revendication 22 ou 23, dans lequel l'ensemble de ressort (22, 102) est constitué, en outre, d'un ressort sinusoïdal (58, 60) ayant une première boucle (66), une dernière boucle (68) et une pluralité de boucles (70), la première boucle (66) définissant la première extrémité en porte-à-faux, la dernière boucle (68) définissant la deuxième extrémité en porte-à-faux et la pluralité de boucles (70) définissant la section centrale, dans lequel la pluralité de boucles (70) est constituée, en outre, d'une deuxième boucle (72) et d'une deuxième-dernière boucle (74), la deuxième boucle (72) étant formée de façon solidaire avec la première boucle (66) et la deuxième-dernière boucle (74) étant formée de façon solidaire avec la dernière boucle (68).

25. Dispositif de support de lombaire selon l'une quelconque des revendications 1 à 3, dans lequel l'ensemble de ressort (22) comprend un premier ressort (58) et un deuxième ressort (60) et une paire de connecteurs (62, 64), la paire de connecteurs (62, 64) fixant le premier ressort (58) au deuxième ressort (60).

26. Dispositif de support de lombaire selon l'une quelconque des revendications 1 à 3, dans lequel la section centrale de l'ensemble de ressort (22) comprend, en outre, une partie évidée (44).

27. Dispositif de support de lombaire selon l'une quelconque des revendications 5 à 18, dans lequel la deuxième monture (126, 206, 308) est configurée afin que la deuxième extrémité de l'ensemble de ressort (122, 202, 302) soit autorisée à réaliser une translation par rapport à la deuxième monture (124, 206, 308) dans une direction vers la première monture (124, 204, 304) lorsque l'ensemble de ressort (122, 202, 302) augmente en courbure ou la trajectoire longitudinale est initialement courbée par l'intermédiaire de l'actionneur.

28. Dispositif de support de lombaire selon l'une quelconque des revendications 5 à 18, dans lequel la deuxième monture (308) comprend, en outre, une paire de ressorts hélicoïdaux (310).
